**Europäisches Patentamt**

**European Patent Office**

**Office européen des brevets**

(11) Veröffentlichungsnummer: **0 043 137**
**B1**

(12) **EUROPÄISCHE PATENTSCHRIFT**

(45) Veröffentlichungstag der Patentschrift: **21.01.87**

(21) Anmeldenummer: **81105064.0**

(22) Anmeldetag: **30.06.81**

(51) Int. Cl.⁴: **G 01 N 33/10, G 01 N 27/22**

(54) **Verfahren und Vorrichtung zur kontinuierlichen Bestimmung der Feuchtigkeit von schüttfähigen Nahrungsmitteln.**

(30) Priorität: **30.06.80 DE 3024794**
**05.12.80 PCt/ch80/00150**

(43) Veröffentlichungstag der Anmeldung:
**06.01.82 Patentblatt 82/01**

(45) Bekanntmachung des Hinweises auf die
Patenterteilung:
**21.01.87 Patentblatt 87/04**

(84) Benannte Vertragsstaaten:
**BE CH DE FR GB IT LI NL SE**

(56) Entgegenhaltungen:
**FR-A-2 345 720**
**GB-A- 597 800**
**SU-A- 173 990**
**US-A-3 144 029**
**US-A-4 168 466**

(73) Patentinhaber: **Gebrüder Bühler AG**
**CH-9240 Uzwil (CH)**

(72) Erfinder: **Oetiker, Hans**
**Sahlistrasse 4**
**CH-9000 St. Gallen (CH)**
Erfinder: **Kummer, Emanuel**
**Bachstrasse 40**
**CH-9202 Gossau (CH)**

(74) Vertreter: **Kehl, Günther, Dipl.-Phys. et al**
**Patentanwälte GEYER, HAGEMANN & KEHL**
**Ismaninger Strasse 108 Postfach 86 03 29**
**D-8000 München 86 (DE)**

Courier Press, Leamington Spa, England.

## Beschreibung

Die Erfindung bezieht sich auf ein Verfahren zur kontinuierlichen kapazitiven Bestimmung der Feuchtigkeit eines einen Gutstrom bildenden rieselfähigen Nahrungsmittels im Durchlaufverfahren, insbesondere eines Nahrungsmittels, dessen Gutanteile inhomogen gemischt sind, bei welchem der Gutstrom in einen Meßstrom und in einen Bypasstrom geteilt wird, der Meßstrom kontinuierlich durch eine als elektrischer Kondensator ausgebildete, eine Eingangs- sowie eine hierzu kleinere Ausgangsöffnung aufweisende Meßzelle geführt wird, derart, daß er unter Bildung eines Rückstaus in der Meßzelle diese infolge der Schwerkraft durchrieselt, hierbei die durch den Meßstrom hervorgerufene Kapizitätsänderung des Kondensators kontinuierlich gemessen wird, die beiden Ströme hinter der Meßzelle wieder zusammengeführt werden und die Gutfeuchtigkeit aus der gemessenen Kapazitätsänderung ermittelt wird.

Die Erfindung betrifft ferner eine Vorrichtung zur kontinuierlichen kapazitiven Bestimmung der Fuchtigkeit eines einen Gutstrom bildenden rieselfähigen Nahrungsmittels im Durchlaufverfahren, insbesondere eines Nahrungsmittels, dessen Gutanteile inhomogen gemischt sind, mit einem im Wege des Gutstromes angeordneten, als Kondensator ausgebildeten Durchlauf-Meßgefäß, dessen Ausgangsöffnung kleiner als dessen Eingangsöffnung ist und das derart angeordnet und ausgebildet ist, daß ein einen Meßstrom bildender Teil des Gutstroms unter Bildung eines das Durchlauf-Meßgefäß füllenden Rückstaus aufgrund der Schwerkraft durch das Durchlauf-Meßgefäß hindurchrieselt, während der restliche Teil des Gutstroms als Bypasstrom am Meßgefäß vorbeigeführt und hinter diesem mit dem daraus austretenden Meßstrom vereinigt wird, einer Einrichtung zur Messung der durch den Meßstrom hervorgerufenen Kapazitätsänderung des Kondensators und einer Signalverarbeitungselektronik zur Ermittlung der Gutfeuchtigkeit aus der Kapazitätsänderung.

## Zugrundeliegender Stand der Technik

Aus der US—A—4 168 464 sind ein Verfahren und eine Vorrichtung der oben genannten Art bekannt. Bei dem bekannten Verfahren bzw. der bekannten Vorrichtung wird das über ein Förderband laufend angeförderte Gut teilweise in einen Probeentnahmetrichter eingeleitet, der zu einer Testzelle führt. Ein Großteil des Gutes fließt jedoch über den zur Testzelle führenden Trichter hinaus und fällt direkt in einen weiteren Sammeltrichter, in dem auch das aus der Testzelle austretende Material eingespeist wird. Die Testzelle weist ausgangsseitig eine konische Verengung auf, so daß eine konstante Füllung dieser Testzelle gewährleistet ist. Die Testzelle ist als zylindrischer Kondensator ausgebildet, dessen Kapazitätsänderung laufend gemessen wird. Bei diesem bekannten Verfahren bzw. der bekannten Vorrichtung wird zwar ein repräsentativer Querschnitt des Gutstromes der Testzelle zugeführt. Zwischen dem Hauptstrom, der an der Testzelle vorbeigeführt wird und dem Meßstrom, der durch die Testzelle durchgeführt wird, entsteht jedoch eine seitliche Verschiebung derart, daß Gutteilchen im Hauptstrom schon sehr viel weiter auf ihrem Strömungsweg fortgeschritten sind, während entsprechende aus der Nähe dieser Teilchen entnommene Probeteilchen sich erst durch die Testzelle bewegen. Diese Verzögerung zwischen den beiden Teilströmen ist bei dem bekannten Verfahren bzw. bei der bekannten Vorrichtung unterschiedlich je nach Durchsatzmenge des Gesamtstromes. Somit kann beim bekannten Verfahren nicht von einer konstanten Verzögerung zwischen den beiden Strömen ausgegangen werden, so daß es schwierig ist, beispielsweise eine nachgeschaltete Netzeinrichtung entsprechend den aus dem Meßkondensator abgeleiteten Meßwerten präzise zu steuern.

Bei dem aus der GB—A—597 800 bekannten Verfahren bzw. der aus dieser Druckschrift bekannten Vorrichtung wird ein Gutstrom nach dem Prinzip aufgeteilt, im Meßgefäß stets ein konstantes Gewicht zu haben. Dies hat den nachteil, daß hierzu eine aufwendige Wägevorrichtung vorgesehen werden muß. Hinzu kommt, daß wegen sich bezüglich ihres Gewichtes ändernden Mischungsanteilen des Gutstromes laufend unterschiedliche Füllhöhen im Meßbehälter vorhanden sind. Unterschiedliche Füllhöhen bringen aber unterschiedliche Kapizitäten. Unterschiedliche Gewichte der Mischungsanteile Können beispielsweise durch unterschiedliche Getreidesorten in den Mischungsanteilen bedingt sein; ferner dadurch, daß Mischungsanteile unterschiedliche Feuchtigkeitsgehalte haben, mit der Folge, daß ein sehr feuchter Mischungsanteil zu einer geringeren Füllhöhe im Meßbehälter führt und dadurch eine geringere Meßfeuchtigkeit vortäuscht. Ein unterschiedliches Gewicht bei den Mischungsanteilen kann auch dadurch hervorgerufen werden, daß die Mischungsanteile unterschiedliche Mengen an Verunreinigungen, insbesondere Steine, enthalten. Hinzu kommt, daß die Füllhöhe des Meßstroms im Meßbehälter bei sich ändernden Durchsatzmengen des Gutstroms laufend um einen Wert pendelt und allein hierdurch Instabilitäten entstehen. Zwar lassen sich derartige Instabilitäten durch die in der Druckschrift beschriebenen Dämpfungsmittel weitgehend unterdrücken, vollständig können derartige Schwankungen aber nicht aufgehoben werden.

Aus der SU—A—173 990 ist ein Feuchtigkeitsmesser für die kapazitive Feuchtigkeitsüberwachung von Isolationsstoffen bekannt, bei denen die Ladung eines Meßkondensators zyklisch auf einen Referenzkondensator umgeladen und dann die Spannung gemessen wird.

Aus der US—A—3 144 029 ist eine Vorrichtung zur Beeinflussung der Feuchtigkeit von Getreide bekannt. Bei dieser bekannten Vorrichtung wird die zur Erzielung eines vorgegebenen Feuchtigkeitssollwertes fehlende Wassermenge ermittelt und der ermittelte Wert als Steuersignal für eine Netzeinrichtung verwendet. Die Feuchtigkeit des Getreides wird durch Messung der elektrischen Leitfähigkeit des Gutstromes bestimmt.

Offenbarung der Erfindung

Der Erfindung liegt die Aufgabe zugrunde, ein Verfahren und eine Vorrichtung der eingangs genannten Art zu schaffen, bei dem die Feuchtigkeitsmeßwerte möglichst exakt zur Verfügung gestellt werden Können und eine möglichst präzise Steuerung einer nachgeschalteten Netzeinrichtung ermöglicht ist.

In verfahrensmäßiger Hinsicht ist die Lösung dieser Aufgabe dadurch gekennzeichnet, daß der Gutstromdurchsatz im Bypasstrom auf eine im wesentlichen konstante Steighöhe in einer den Bypasstrom führenden Bypassleitung geregelt wird.

In vorrichtungsmäßiger Hinsicht ist die Lösung dieser Aufgabe durch eine Niveauregeleinrichtung zur Regelung der Durchsatzleistung des Bypasstromes auf eine konstante Steighöhe in einem den Bypasstrom führenden Bypasskanal, wleche einen Istwertfühler und ein Stellorgan umfaßt, gekennzeichnet.

Durch die erfindungsgemäße Niveauregelung wird ein konstanter Gutrückstau und somit reproduzierbare Verhältnisse bei der Aufteilung des Gutstromes in die beiden Teilströme sichergestellt.

Die Erfindung wird im folgenden anhand der in den Figuren schematisch dargestellten Ausführungsbeispiele näher erläutert.

Kurze Beschreibung der Zeichnungen

Es zeigen:

Die Figur 1 die Messung der Feuchtigkeit und Kontrolle der Durchflußgeschwindigkeit durch eine Meßstrecke; direkt unter einem Silo;

die Figur 2 eine diagrammatische Darstellung des elektrischen Meßverfahrens;

die Figur 3 die Stellung einzelner Schalter im zeitlichen Verlauf gemäß Figur 2;

die Figur 4 ein Prinzipschema für die Steuerung und Regelung einer Getreidenetzeinrichtung;

die Figur 5 die Verwendung der Lösung gemäß Figur 4 in einer Mühle oder Putzerei;

die Figur 6 die Messung der Feuchtigkeitund der Durchflußmenge direkt am Produktstrom;

die Figur 7 schematisch die Durchschnittsbildung des Gutes in dem Meßbehälter.

Wege zur Ausführung der Erfindung

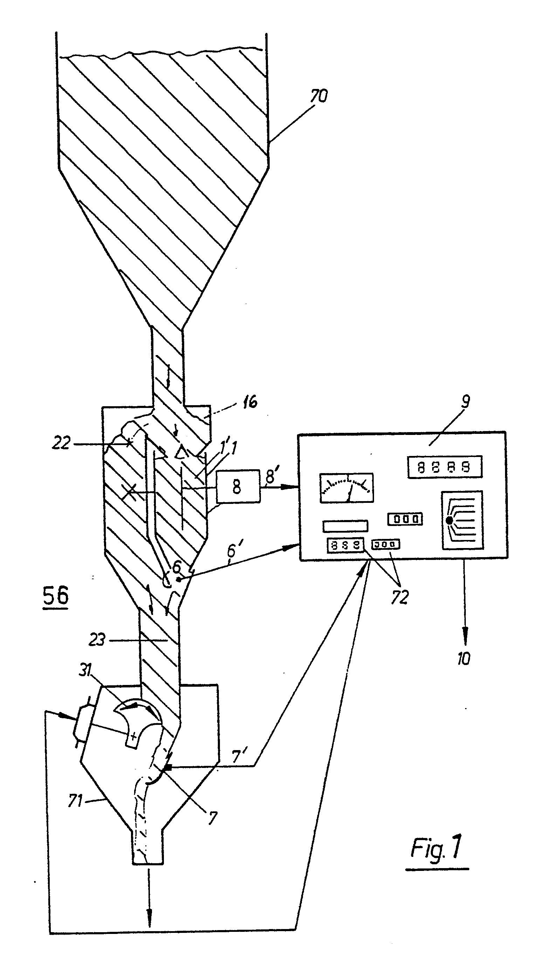

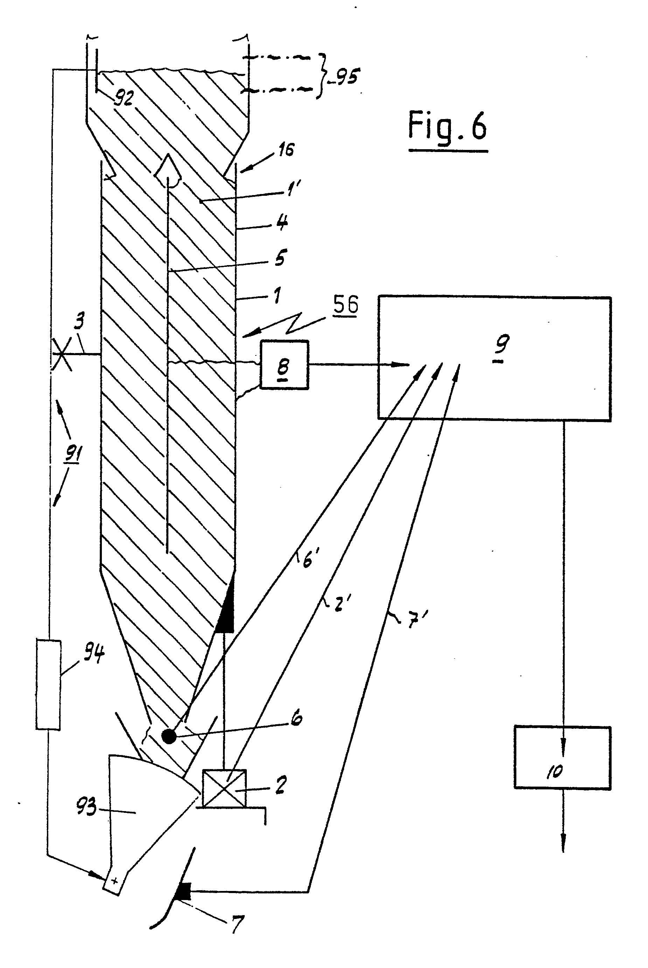

Die Figur 1 zeigt eine Prinzipdarstellung einer unter einer Silozelle 70 angeordneten Vorrichtung 56 zur kontinuierlichen Bestimmung der Feuchtigkeit von Getreide, wobei die Getreidebewegung im wesentlichen ausschließlich durch die Schwerkraft und die Formgebung der Vorrichtung 56 bestimmt wird. Die Vorrichtung 56 weist ein sich an den Auslaß der Silozelle 70 anschließendes Meßgehäuse 16 auf, das sich an seinem stromabwärtigen Ende zu einem Produktauslaß 23 verjüngt. An den Produktauslaß 23 schließt sich stromabwärts ein Durchflußregelkreis 71 an. Das Meßgehäuse 16 ist in einen Regelkanal 22 und einen Meßbehälter 1 unterteilt. Das Meßgehäuse 16 ist derart angeordnet und ausgelegt, daß die Längsachsen des Regelkanales 22 und des Meßbehälters 1 im wesentlichen in Richtung des Schwerkraftfeldes der Erde, also vertikal verlaufen und das Schüttgut quasi durchrieselt, ähnlich wie durch eine Sanduhr. Der Meßbehälter 1 weist eine Schüttgutmeßkanal- beziehungsweise -strecke 1' auf, die zur kapazitiven Messung der Getreidefeuchtigkeit als Kondensator ausgebildet ist. Wie in Figur 6 vergrößert dargestellt, ist hierzu eine Behälterwandung des Meßbehälters 1 als eine erste Kondensatorplatte 4 ausgebildet und eine zweite Kondensatorplatte 5 im Behälterinneren angeordnet. Unter dem Meßbehälter 1 ist ein Schüttguttemperaturmeßfühler 6 angeordnet. Unterhalb des Produktauslasses 23 befindet sich ein Durchflußmengenmeßgerät 7. Die Meßwerte werden teils in einer Wandlereinheit 8 sowie einer Auswertelektronik 9 zur gewünschten Kenngrösse aufgearbeitet. Die Ausgangswerte des Schüttguttemperaturfühlers 6, des Durchflußmengenmeßgerätes 7 und der Wandlereinheit 8 werden Über Signalleitung 6', 7' beziehungsweise 8' der Auswertelektronik 9 zugeführt.

Die in der Figur 1 dargestellte Lösung ist im Prinzip die Grundlage für die Messung der Feuchtigkeit von Getreide und kann — ergänzt mit dem genannten Durchflußmengenmeßgerät 7 sowie einem (zweiten) Rechner 10 (siehe Figur 6) — direkt zur Steuerung der Aufnetzung, das heißt Beeinflussung des Feuchtigkeitsgehaltes des Getreides auf einen bestimmten Wert benützt werden.

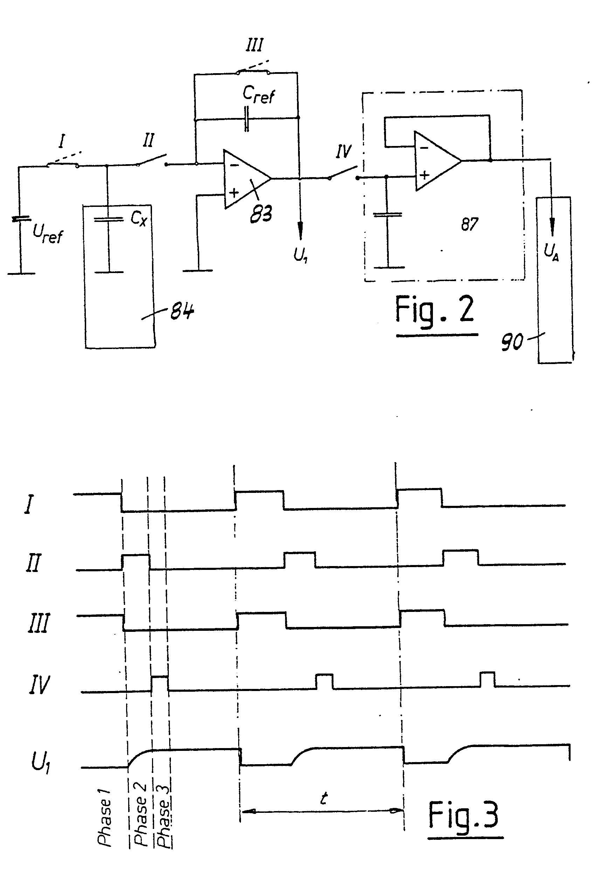

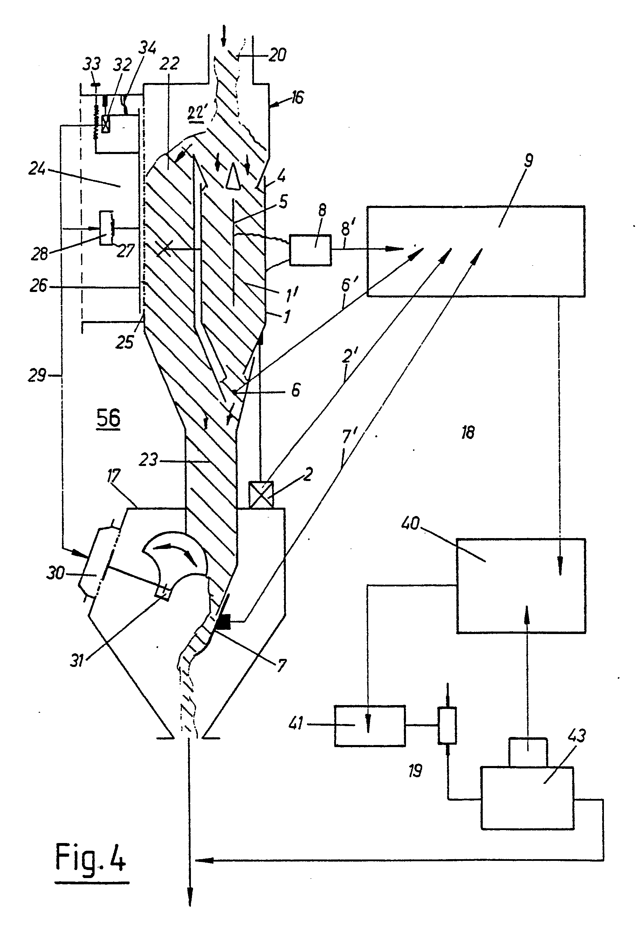

In der Figur 2 ist nun in einer Prinzipdarstellung das neue Meßverfahren dargestellt. Hierbei wird die Kapazität einer als Kondensator ausgebildeten Meßstrecke 84, sinngemäß zur Schüttgutmeßstrecke 1' der Figur 1 (sowie Figuren 4, 5, 6, 7) gemessen. Für den Ablauf der Messung wird gleichzeitig auch auf die Figuren 3 und 1 Bezug genommen.

Die Figur 3 zeigt jeweils die einzelnen Schalterstellungen von Schaltern I—IV in Figur 2 während der Messung und den Verlauf einer Spannung $U_1$, welche der elektrischen Ladung eines Referenzkondensators $C_{ref}$ zugeordnet ist.

In einer ersten Phase wird ein Meßkondensator $C_x$ der Meßstrecke 84 mit einer vorbestimmten Spannung $U_{ref}$ aufgeladen. Gleichzeitig wird der Referenzkondensator $C_{ref}$ entladen. Schalter I und III sind geschlossen. Schalter II und IV sind geöffnet (Position wie in Figur 2). Die Spannung $U_1$ ist in der ersten Phase noch gleich Null. In einer zweiten Phase wird die Ladung des Meßkondensators $C_x$ auf den Referenzkondensator $C_{ref}$ umgeladen. Der Schalter II wird dazu geschlossen und die Schalter I, III und IV sind geöffnet. Die Spannung $U_1$ steigt solange, bis der Meßkondensator $C_x$ völlig entladen ist. Diese Umladung wird mit Hilfe eines Operationsverstärkers 83 bewerkstelligt. In einer dritten Phase wird der Endwert

der Spannung $U_1$ auf einen Analogspeicher 87 übergeben. Zu diesem Zwecke wird der Schalter IV geschlossen und werden die Schalter I—III geöffnet. Dieses phasenweise Auf- und Entladen von Meß- und Referenzkondensator $C_x$ bzw. $C_{ref}$ wird vorzugsweise durch den Zyklus der Netzfrequenz bestimmt. Aus dem Analogspeicher 87 wird eine Arbeitsspannung $U_a$ über eine Schnittstelle (Interface) 90 auf eine nachgeschaltete Steuereinrichtung, zum Beispiel zur Steuerung der Aufnetzung (Wasserzugabe), gegeben.

Die Figur 6 zeigt ein besonders vorteilhaftes Ausführungsbeispiel, das auf den Grundelementen der Figuren 1, 2 und 3 aufgebaut ist und insbesondere für kleinere Produktleistungen gedacht ist. Gleiche Teile sind deshalb mit gleichen Bezugszeichen versehen. In Figur 6 ist zusätzlich eine Steuereinrichtung 91 zur Steuerung der Senkbewegung des Gutes in der Schüttgutmeßstrecke 1', beziehungsweise im Meßbehälter 1 vorgesehen. Die Steuereinrichtung 91 weist eine Niveausonde 92 sowie einen Einstellschieber 93 auf. Die Signale der Niveausonde 92 werden von einem Kommandogerät 94 verarbeitet.

Die Aufgabe der Steuereinrichtung 91 besteht darin, daß das Produkt, verzögert durch die Schüttgutmeßstrecke 1' beziehungsweise den Meßbehälter 1 geführt wird. Die Steuereinrichtung 91 sorgt dafür, daß die Produktbewegung mehr oder weniger entsprechend dem Gutdurchsatz verzögert wird, so daß der Meßbehälter 1 ständig mit dem Gut gefüllt ist. Zur Sicherstellung der Füllung wird mit der Steuereinrichtung 91, durch Steuerung der Offenstellung des Einstellschiebers 93 das Gut in einem konstanten Niveaubereich 95 gehalten.

Durch den geregelten Rückstrom wird sichergestellt, daß auch bei abgestelltem Gutstrom keine Fehlanzeige oder Fehlbefehle an die Arbeitselemente (Wasserventil und so weiter) abgegeben werden.

Die Figur 6 zeigt auch die weitere Möglichkeit der Erfassung der Schüttungsdichte von dem Gut durch eine Wägeeinrichtung, bestehend aus einer Druckmeßdose beziehungsweise Waage 2 sowie einer entsprechenden Gelenkaufhängung 3. Zu diesem Beispiel ist der Meßbehälter 1 gleichzeitig als Waagebehälter ausgebildet. Das Ausgangssignal der Waage 2 wird über eine Signalleitung 2' der Auswertelektronik 9 zugeführt.

Es hat sich gezeigt, daß bei gewissen Produkten besonderes im Hinblick auf deren elektrisches Leit- oder Polarisationsverhalten, die zusätzliche Wägung und Verwertung des Gewichtsmeßwertes, der vorteilhafterweise gleichzeitig wie der elektrische Wert festgestellt wird, eine erhöhte Sicherheite für das Meßergebnis ergibt. Besonders hervorzuheben ist noch, daß sich interessanterweise gezeigt hat, daß in sehr vielen Anwendungsfällen der Erfindung auch ohne Gewichtsbeziehungsweise Schüttdichtemessung äußerst repräsentative Werte erhalten werden.

Die Auswertelektronik 9 kann nun auch so programmiert sein, daß zum Beispiel der Gewichtswert ständig gemessen, solange er aber im Bereich der bekannten zulässigen Grenzen liegt, in der Auswertung nicht berücksichtigt wird. Diese Lösung kann zum Beispiel dann wertvoll sein, wenn normalerweise die Feuchtigkeit von rohem Weizen gemessen wird. Ist nun zum Beispiel der Weizen ungenügend gereinigt, oder hat in der vorangehenden Silozelle eine starke Entmischung stattgefunden, so daß das letzte austretende Gut mehr aus Staub und Schmutz, und so weiter, als aus Weizen besteht, so würde in diesem Fall wegen dem großen Anteil an toten Schalenteilen und mineralischen Bestandteilen die angezeigte Kenngröße, zum Beispiel die Feuchtigkeit, falsch sein.

Die Mitberücksichtigung der Dichte kann einerseits als eine Sicherheit, oder aber im Falle von tatsächlich großen Schwankungen in der Dichte zur Korrektur des Meßwertes verwendet werden.

In Figur 4 wird eine Anwendung der Erfindung zur Messung und Regulierung des Wassergehaltes eines Schüttgutes dargestellt. Die Vorrichtung 56 weist das Meßgehäuse 16, ein Durchflußregelorgan 17 und einen Regel- und Steuerkreis 18 auf, der die Auswertelektronik 9 und einen Netzapparat 19 beinhaltet. Gleiche Teile in Figur 1 und 4 sind mit gleicher Bezugsnummer versehen.

Das Meßgehäuse 16 weist einen Einlauf 20 auf, der im wesentlichen senkrecht über dem Meßbehälter 1 einmündet.

Wie in Figur 1 verlaufen der Meßbehälter 1 und der Regelkanal 22 parallel zueinander, sind unterhalb des Einlaufs 20 durch einen Überleitkanal 22' verbunden und führen im Bereich oberhalb des Produktauslasses 23 zusammen. Im Überleitkanal 22' verteilt sich das Produkt freifließend auf den Regelkanal 22 und die Meßstrecke 1'. Der Produktauslaß 23 wird über das Durchflußregelorgan 17 gesteuert, indem ein Niveaufühler 24 als seitlich am Regelkanal 22 angeordnete Membran 25 ausgebildet ist.

Eine Versteifung 26 ist drehbar in einem Gelenk 34 befestigt. Ebenso ist ein pneumatisches Regulierventil 32 mit der Versteifung 26 verbunden. Bei einem bestimmten Produktdruck wird über die Membran 25 und die Versteifung 26 das pneumatische Regulierventil 32 betätigt, welches einen Luftdruck in eine Leitung 29 gibt. Mit diesem Luftdruck wird eine Druckeinrichtung 30 betätigt, welche ihrerseits einen Schieber 31 beim Produktauslaß 23 reguliert. Der gleiche Druck, der in der Leitung 29 ist, kommt auch in eine Druckkammer 28 und wirkt auf eine Membran 27. Hier wirkt der Druck als Kompensationsdruck zum Produktdruck.

Am Regelkanal 22 befinden sich ferner Handbetätigungsmittel für das Öffnen und Schließen des Durchflußregelorgans 17. Über eine Schraube 33 und die Versteifung 26 läßt sich das pneumatische Regulierventil 32 betätigen, und somit der Schieber 31 vollständig öffnen oder schließen. Mit diesen Handbetätigungsmitteln ist es möglich, das Meßgehäuse 16 völlig zu entleeren, um zum Beispiel eine Kontrolle des Meßkonden-

sators $C_x$ beziehungsweise der Meßstrecke 1' durchzuführen.

Unterhalb des Produktauslasses 23 des Meßgehäuses 16 ist wiederum das Durchsatzmengenmeßgerät 7 angeordnet, welches die momentane Durchsatzmenge feststellt. Die Meßwerte des Kondensators $C_x$ beziehungsweise 4, 5 des Schüttguttemperaturmeßfühlers 6, der Waage 2 und des Durchsatzmengenmeßgerätes 7 werden über die Signalleitungen 8', 6', 2' beziehungsweise 7' der Auswertelektronik 9 zugeführt, die daraus eine Fehlwassermenge ermittelt und den ermittelten Wert in Form einer Steuergröße einem Regler 40 übergibt. Letzterer steuert einen Motor 41, welcher die errechnete Fehlwassermenge in den Produktfluß überführt. Als Kontrolle wird noch einmal durch eine Zwischenstelle 43 die weggehende Wassermenge rückgemeldet.

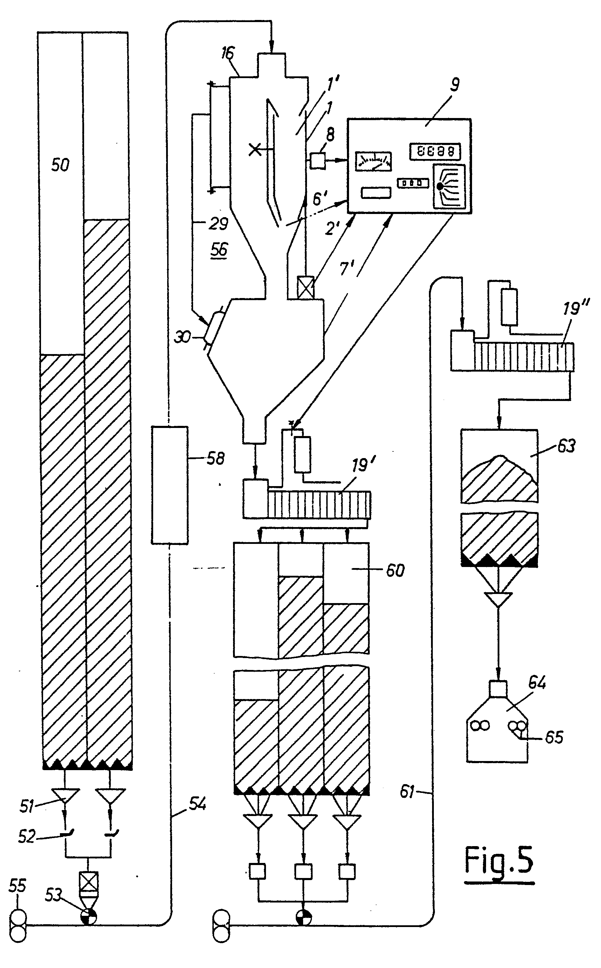

In Figur 5 sind die zu Figur 4 gleichbleibenden Teile mit der gleichen Bezugsziffer versehen. Figur 5 zeigt ein Grundschema einer mit einer neuen Meßvorrichtung 56 beziehungsweise nach dem neuen Meßverfahren arbeitenden Anlage.

Die Rohfrucht wird aus Lagerzellen 50 über spezielle Ausläufe 51, welche ein Entmischen verhindern, abgezogen. Mittels eines Schiebers 52 wird eine ungefähre Austragsleistung eingestellt. Über eine Zellenradschleuse 53 wird die ausgetragene Rohfrucht mittels der durch ein Gebläse 55 erzeugten Luft über einen pneumatischen Förderstrang 54 in die Feuchtigkeitsmeßvorrichtung 56 gefördert. Ein wesentlicher Punkt in der Anwendung von Figur 5 liegt darin, daß das Gut über eine größere Anzahl Reinigungsmaschinen der Putzerei 58 geführt wird. Dabei werden Leistungsschwankungen und auch Gutmischungen erzeugt, so daß für die anschließende Feuchtigkeitsmessung erschwerte Bedingungen gegeben sind.

Die baulichen Einzelheiten des Meßbehälters 1 sind bereits in Figur 4 dargestellt. Ein Intensivnetzapparat 19' weist in der vorteilhaften Lösung ein geschlossenes Gehänge mit einem schnell umlaufenden Intensivnetzrotor auf. Der Wassergehalt des Getreides wird im Meßbehälter 1 festgestellt und anschließend in einem offenen beziehungsweise Vorwärtsregelkreis (Steuerkette) über die Auswertelektronik 9 und den Intensivnetzapparat 19' gesteuert. Der Intensivnetzapparat 19' ist in der Zusammenwirkung mit dem erfindungsgemäßen Meßverfahren deshalb besonders vorteilhaft, weil die Gewinnung der momentanen Feuchtigkeitssignale sowie die Steuerung der entsprechenden Netzwasserzugabe in Sekundenschnelle durchgeführt wird.

Das aufgenetzte Getreide wird in Abstehzellen 60 gefördert. Je nach Weizensorte und erforderlichen Mahlprodukten kann das Korn nach einer Anzahl Stunden den Abstehzellen 60 entnommen und über einen weiteren pneumatischen Transport 61 in eine weitere Intensivnetzvorrichtung 19" gegeben werden, wo noch eine geringe Restwassermenge zugegeben wird. Meistens werden hier einige Zahntel Prozent Wasser als Wasserfilm auf das Korn aufgetragen, und das Korn nach einer Einwirkzeit in Abstehkästen 63 direkt Walzenstühlen 64 zugeführt. Zwischen den Walzen 65 der Walzenstühle 64 wird das Korn dann in bekannter Weise zu Gries oder Mehl vermahlen.

In Figur 1 ist noch eine weitere Möglichkeit der Anwendung der neuen Erfindung gezeigt. Die Vorrichtung ist direkt unterhalb eines Silos angebracht. Das Produkt fließt von der Silozelle 70 direkt durch das Meßgehäuse 16, welches in den die Meßstrecke 1' aufweisenden Meßbehälter 1 und den Regelkanal 22 unterteilt ist.

Beim Produktauslaß 23 befindet sich der Durchflußregelkreis 71, der entsprechend dem vorgegebenen Wert eines Sollwertgebers 72 in der Auswertelektronik 9 die Gutdurchflußmenge steuert. Der Durchflußregelkreis 71 weist ein Durchflußmengenmeßgerät 7 (Istwertfühler) und den steuerbaren Schieber 31 auf. In der Auswertelektronik 9 werden der Istwert und der Sollwert der Durchflußmenge miteinander verglichen. Das durch diesen Vergleich erhaltene Fehlersignal wird zur Steuerung des Schiebers 31 verwendet. Die Auswertelektronik 9 dient also insoweit als Komparator. Zusätzlich werden in der Auswertelektronik 9 der Wassergehalt und die Durchflußmenge miteinander verknüpft und mittels eines aus dieser Verknüpfung gewonnenen Steuersignales die Aufnetzung oder Trocknung gesteuert (siehe zum Beispiel Figur 4).

Die erfindungsgemäße Vorrichtung erhält in der dargestellten Anwendung eine Doppelfunktion: einerseits wird der genaue Wassergehalt des Schüttgutes ermittelt, und anderseits wird eine exakte Austragleistung aus der Silozelle 70 erreicht.

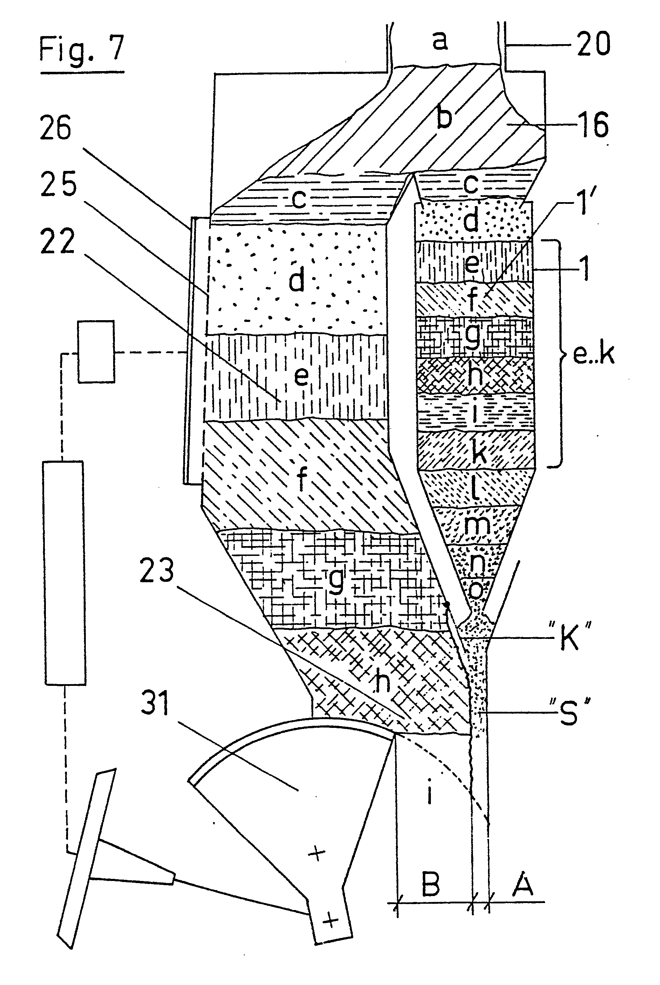

Die Figur 7 zeigt in etwas vergrößertem Maßstab die kontinuierliche Durchschnittsbildung des Gutes in der in Figur 1 und Figur 4 gezeigten Meßstrecke 1' im Meßbehälter 1.

Diese kontinuierliche Durchschnittsbildung, das heißt kontinuierliche Bildung eines stets repräsentativen Querschnittes des den Einlauf 20 durchströmenden Schüttgutes, stellt sich in der erfindungsgemäßen Vorrichtung 56 stets von selbst ein.

Es werde im folgenden angenommen, daß sich die Zusammensetzung des Gutes, das heißt die einzelnen Gutanteile, über die Zeit gesehen ändert. Zur Veranschaulichung ist eine chargenweise oder postenweise Änderung dargestellt worden. Der Einfluß der Durchschnittsbildung kann jedoch auf die genau gleiche Weise erzielt werden, wenn die Änderung allmählich ist.

Das Gut wird bei dem Einlauf 20 eingespeist und bewegt sich nur mittels Schwerkraft in dem Regelkanal 22 sowie in dem Meßbehälter 1 respektive Meßstrecke 1' nach unten. Im Regelkanal 22 wird die Gutbewegung durch den Schieber 31 verzögert, so daß dort übereinandergeschichtet die verschiedenen Gutanteile c, d und e bis h zu liegen kommen. Der Gutanteil a befindet sich noch in dem Einlauf 20, der Gutanteil b ist im Begriffe sich auf die beiden Wege (Regelkanal 22—Meßstrecke 1') aufzuteilen. Der Gutanteil i verläßt den Regelkanal 22 im Bereich des Schiebers 31. Wenn auch das Gut aufgestaut wird bis auf die Höhe der Membrane 25, so ergibt sich in dem Regelkanal 22 doch eine der Durchsatzleistung sowie des Regelkanalquerschnittes entsprechend große Durchtrittsgeschwindigkeit.

Anders liegen die Verhältnisse in der Meßstrecke 1'. Hier wird durch Formgebung des Meßbehälters 1, besonders durch die Querschnittsverhältnisse im Bereich der elektrischen Messung, das heißt der Meßstrecke 1', sowie der verengten Austrittsöffnung des Meßbehälters 1, die durch die Schwerkraft bewirkte Senkbewegung des Gutes viel stärker verzögert. Demgemäß befinden sich Gutanteile e, f, g, h, i und k zwar noch in der Meßstrecke 1', nicht mehr dagegen in dem der Meßstrecke 1' seitlich benachbarten Regelkanal 22. Der eigentliche Gutinhalt in der Meßstrecke 1' kann eine Größenordnung von 5 Liter bis zu 20 Liter und mehr haben. In dem Bereich direkt oberhalb des Schiebers 31 findet ein Geschwindigkeitsausgleich statt zwischen der relativ großen Bewegung des aus dem Regelkanal 22 austretenden Gutes sowie der vergleichsweise geringeren Gutbewegung aus der Meßstrecke 1'. Beim Meßbehälter 1 ist dabei die unterste Öffnung "S" maßgebend, welche zudem durch eine Klappe "K" vorwählbar ist. Aus dem Geschilderten ist ersichtlich, daß die Senkbewegung in der Meßstrecke 1' um ein Vielfaches kleiner ist als die Senkbewegung in dem Regelkanal 22, entsprechend etwa den beiden Gutstromquerschnitten "A" und "B", wenn die oberen Querschnitte des Regelkanals 22 und der Meßstrecke 1' im Bereich der Membrane 25 etwa gleich sind. Je kleiner der Spalt "S" mit der Klappe "K" eingestellt wird, umso kleiner ist die Senkbewegung des Gutes in der Meßstrecke 1', umso größer die Verweilzeit des Gutes in der Meßstrecke 1', umso kleiner jedoch wieder das Volumen jedes einzelnen Gutanteiles in der Meßstrecke 1', umso größer die Anzahl der variierenden Gutanteile in der Meßstrecke 13 und als Folge umso ausgeprägter Gutdurchschnittsbildung.

Die elektrische Messung gemäß Figur 7 umfaßt deshalb einen Durchschnitt (Mittelwert) aus den Gutanteilen e—k.

Die effektive Messung läuft auf diese Weise dem Gutstrom etwas nach, was jedoch besonders mit der Durchschnittsbildung ein weiterer Vorteil ist, da eine allenfalls notwendige Wasserzugabe — bis sie in den nachfolgenden Apparaten wirksam ist — eine gewisse Zeit braucht. Der Spalt "S" und die Gesamtdurchtrittsleistung des Gutes durch den Schieber 31 können so aufeinander abgestimmt werden, daß entweder die maximal mögliche Genauigkeit der Wasserzugabe oder eine optimale Befeuchtung der Menge des Gutes erreicht wird und dabei alle Vorteile der Vorwärtsregelung (Steuerung) der Feuchtigkeitszugabe genutzt werden können, welche hier vorzugsweise zur Anwendung kommt.

Aus den vorangegangenen Schilderungen ergibt sich, daß die Erfindung tatsächlich eine wesentliche Verbesserung der Messung der Feuchtigkeit und der Steuerung der notwendigen Wasserzugabe ergibt. Das elektrische Meßverfahren, insbesondere das kapazitive Meßverfahren hat den Vorteil, daß der ganze Inhalt der Meßstrecke, zum Beispiel 5—20 Liter kontinuierlich gemessen wird. Die erwähnten 5—20 Liter stellen jedoch wegen der geschilderten Gutverzögerung in der Meßstrecke 1' einen Durchschnitte von 50, 100 oder mehr Liter das Meßgerät durchströmenden Getreides dar. Dies bedeutet, daß die Feuchtigkeitsmessung ständig Getreide in der Größenordnung eines Sackes mißt und damit repräsentative Resultate anbietet.

**Patentansprüche**

1. Verfahren zur kontinuierlichen kapazitiven Bestimmung der Feuchtigkeit eines einen Gutstrom bildenden rieselfähigen Nahrungsmittels im Durchlaufverfahren, insbesondere eines Nahrungsmittels, dessen Gutanteile inhomogen gemischt sind, bei welchem der Gutstrom in einen Meßstrom und in einen Bypasstrom geteilt wird, der Meßstrom kontinuierlich durch eine als elektrischer Kondensator ($C_x$; 4, 5) ausgebildete, eine Eingangs- sowie eine hierzu kleinere Ausgangsöffnung aufweisende Meßzelle (1, 1') geführt wird, derart, daß er unter Bildung eines Rückstaus in der Meßzelle (1, 1') diese infolge der Schwerkraft durchrieselt, hierbei die durch den Meßstrom hervorgerufene Kapizitätsänderung des Kondensators ($C_x$; 4, 5) kontinuierlich gemessen wird, die beiden Ströme hinter der Meßzelle wieder zusammengeführt werden und die Gutfeuchtigkeit aus der gemessenen Kapazitätsänderung ermittelt wird, dadurch gekennzeichnet, daß der Gutdurchsatz im Bypasstrom auf eine im wesentlichen konstante Steighöhe in einer den Bypasstrom führenden Bypassleitung (22) geregelt wird.

2. Verfahren nach Anspruch 1, dadurch gekennzeichnet, daß die Kapazität des Kondensators ($C_x$; 4, 5) in zwei sich zyklisch wiederholenden Phasen derart gemessen wird, daß

a) während einer ersten Phase der Kondensator ($C_x$; 4, 5) mit einer vorgegebenen Spannung ($U_{ref}$) beaufschlagt und ein zusätzlicher Referenzkondensator ($C_{ref}$) entladen wird und

b) während einer sich hieran anschließenden zweiten Phase die Ladung des Kondensators ($C_x$; 4, 5) auf den Referenzkondensator ($C_{ref}$) umgeladen und hierbei dessen Spannung ($U_1$) gemessen wird.

3. Verfahren nach Anspruch 2, dadurch gekennzeichnet, daß die Gutfeuchtigkeit aus dem Mittelwert mehrerer Werte der Spannung ($U_1$) des Referenzkondensators ($C_{ref}$) ermittelt wird.

4. Verfahren nach einem der vorstehenden Ansprüche, dadurch gekennzeichnet, daß das Gut in der Meßzelle (1, 1') gewogen wird.

5. Verfahren anch einem der vorstehenden Ansprüche, dadurch gekennzeichnet, daß die momentane Durchsatzleistung der wieder zusammengeführten beiden Teilströme gemessen wird.

6. Verfahren nach Anspruch 5, dadurch gekennzeichnet, daß aus der ermittelten Gutfeuchtigkeit sowie der momentanen Durchsatzleistung und einem vorgegebenen Feuchtigkeitssollwert die dem Gutstrom zum Erreichen des Feuchtigkeitssollwertes zuzugebende Wassermenge ermittelt und daraus ein Steuersignal für die Steuerung der Zufuhr einer Fehlwassermenge gewonnen wird.

7. Vorrichtung zur kontinuierlichen kapazitiven Bestimmung der Feuchtigkeit eines einen Gutstrom bildenden rieselfähigen Nahrungsmittels im Durchlaufverfahren, insbesondere eines Nahrungsmittels, dessen Gutanteile inhomogen gemischt sind, mit

— einem im Wege des Gutstroms angeordneten, als Kondensator ($C_x$; 4, 5) ausgebildeten Durchlauf-Meßgefäß (1), dessen Ausgangsöffnung kleiner als dessen Eingangsöffnung ist und das derart angeordnet und ausgebildet ist, daß ein einen Meßstrom bildender Teil des Gutstroms unter Bildung eines das Durchlauf-Meßgefäß füllenden Rückstaus aufgrund der Schwerkraft durch das Durchlauf-Meßgefäß hindurchrieselt, während der restliche Teil des Gutstroms als Bypasstrom am Meßgefäß vorbeigeführt und hinter diesem mit dem daraus austretenden Meßstrom vereinigt wird,

— einer Einrichtung (8) zur Messung der durch den Meßstrom hervorgerufenen Kapazitätsänderung des Kondensators ($C_x$; 4, 5),

— einer Signalverarbeitungselektronik (9) zur Ermittlung der Gutfeuchtigkeit aus der Kapazitätsänderung, gekennzeichnet durch eine Niveauregeleinrichtung (24 bis 34) zur Regelung der Durchsatzleistung des Bypasstromes auf eines konstante Steighöhe in einem den Bypasstrom führenden Bypasskanal (22), welche einen Istwertfühler (25) und ein Stellorgan (31) umfaßt.

8. Vorrichtung nach Anspruch 7, dadurch gekennzeichnet, daß das Durchlauf-Meßgefäß (1) und der Bypasskanal (22) unmittelbar nebeneinander derart angeordnet und ausgebildet sind, daß ihre Längsachsen im wesentlichen vertikal und parallel zueinander verlaufen, ihre Eingangsöffnungen über einen gemeinsamen Überlaufkanal (22') verbunden sind und der Bypasskanal (22) über seine gesamte Länge einen größeren Querschnitt als die Ausgangsöffnung des Meßgefäßes (1) hat.

9. Vorrichtung nach wenigstens einem der Ansprüche 7 und 8, dadurch gekennzeichnet, daß die Ausgänge des Meßgefäßes (1) und des Bypasskanals (22) in einen gemeinsamen Gutstromauslaß (23) münden.

10. Vorrichtung nach einem der Ansprüche 7 bis 9, dadurch gekennzeichnet, daß in der Niveau-Regeleinrichtung (24 bis 34) der Istwertfühler eine als Wandabschnitt des Bypasskanals (22) ausgebildete Membran (25) und das Stellorgan ein den Öffnungsquerschnitt des Gutstromauslasses (23) steuerbar änderndes Durchflußregelorgan (31) ist.

11. Vorrichtung nach Anspruch 10, dadurch gekennzeichnet, daß zusätzlich von Hand betätigbare Mittel (33) zum Freigeben oder Verschließen des Gutstromauslasses (23) mittels des Durchflußregelorgans (31) vorgesehen sind.

12. Vorrichtung nach einem der Ansprüche 7 bis 11, gekennzeichnet durch ein Durchsatzmengenmeßgerät (7) zur Ermittlung der momentanen Durchsatzleistung des Gutstromes.

13. Vorrichtung nach einem der ansprüche 7 bis 12, gekennzeichnet durch einen Durchsatzregelkreis (71) zur Regelung eines konstanten Gustrom-Durchsatzes.

14. Vorrichtung nach einem der Ansprüchen 12 oder 13, dadurch gekennzeichnet, daß neben der Kapazitätsmeßeinrichtung (8) auch das Durchsatzmengenmeßgerät (7) mit den Signaleingängen der Signal-Verarbeitungselektronik (9) verbunden ist und die Signal-Verarbeitungselektronik (9) ausgangsseitig an ein einen Komparator enthaltendes Steuerglied (10, 40) zur Steuerung eines stromabwärts des Gutstromauslasses (23) angeordneten Stellgliedes wie z.B. einem Netzapparat (19) ausgelegt ist, in welchem der Gutstrom auf einen vorgegebenen Feuchtigkeitsgehalt gebracht wird.

15. Vorrichtung nach Anspruch 14, dadurch gekennzeichnet, daß das Durchlauf-Meßgefäß (1) als Wägegefäß einer Wägeeinrichtung (2) ausgebildet ist, deren Meßausgang mit dem Eingang der Signal-Verarbeitungselektronik (9) verbunden ist.

16. Vorrichtung nach einem der Ansprüche 7 bis 15, dadurch gekennzeichnet, daß die Außenwandung des Durchlauf-Meßgefäßes (1) als ein erster Belag (4) des Kondensators ausgebildet und ein zweiter Belag (5) im Inneren des Durchlauf-Meßgefäßes (1) angeordnet ist.

## Revendications

1. Procédé de détermination capacitive an continu de l'humidité d'une produit alimentaire susceptible de s'écouler en formant un courant de produit dans le cadre d'un procédé en continu, en particulier d'un produit alimentaire dont les ingrédients sont mélangés de façon hétérogène, dans lequel on divise le courant de produit en un courant de mesure et un courant de dérivation, on fait passer le courant de mesure en continu par un compartiment de mesure (1, 1') réalisé sous la forme d'un condensateur électrique ($C_x$; 4, 5) comportant une ouverture d'entrée, ainsi qu'une ouverture de sortie plus petite que la précédente, de telle façon qu'il s'écoule à travers le compartiment de mesure (1, 1') sous l'effet de la pesanteur en y formant une accumulation, on mesure alors en continu la variation de capacité du condensateur ($C_x$; 4, 5) provoquée par le courant de mesure, on rassemble de nouveau les deux courant après le compartiment de mesure et l'on détermine l'humidité du produit à partir de la variation de capacité mesurée, caractérisé en ce que l'on régle le débit de produit du courant de dérivation à une hauteur de montée sensiblement constante, dans une conduite de dérivation (22) canalisant le courant de dérivation.

2. Procédé selon la revendication 1, caractérisé en ce que l'on mesure la capacité du condensateur ($C_x$; 4, 5) en deux phases se répétant cycliquement de telle façon que:

a) pendant une première phase, on applique au condensateur ($C_x$; 4, 5) une tension ($U_{ref}$) prédéterminée et l'on décharge un condensateur de référence ($C_{ref}$) supplémentaire, et

b) pendant une seconde phase succédant immédiatement à cette première phase, on transfère la charge du condensateur ($C_x$; 4, 5) sur le consensateur de référence ($C_{ref}$) et l'on mesure alors sa tension ($U_1$).

3. Procédé selon la revendication 2, caractérisé en ce qu'on détermine l'humidité du produit à partir de la moyenne de plusieurs valeurs de la tension ($U_1$) du condensateur de référence ($C_{ref}$).

4. Procédé selon l'une des revendications précédentes, caractérisé en ce qu'on pèse le produit dans le compartiment de mesure (1, 1').

5. Procédé selon l'une des revendications précédentes, caractérisé en ce qu'on mesure le débit instantané des deux courants partiels à nouveau rassemblés.

6. Procédé selon la revendication 5, caractérisé en ce qu'on détermine à partir de l'humidité déterminée du produit ainsi que du débit instantané et d'une valeur prescrite de l'humidité prédéterminée, la quantité de chaleur à céder au courant de produit pour obtenir la valeur prescrite de l'humidité, et l'on en tire un signal de commande destiné à commander l'amenée d'une quantité d'eau correctrice.

7. Dispositif de détermination capacitive en continu de l'humidité d'un produit alimentaire susceptible de s'écouler en formant un courant de produit dans le cadre d'un procédé en continu, notamment d'un produit alimentaire dont les ingrédients sont mélangés de façon hétérogène, comportant:

— un compartiment de mesure en continu (1) réalisé sous la forme forme d'un condensateur ($C_x$; 4, 5), placé sur le trajet du courant de produit, dont l'ouverture de sortie est plus petite que son ouverture d'entrée et qui est disposé et conformé de façon qu'une fraction du courant de produit constituant un courant de mesure s'écoule à travers le compartiment de mesure en continu sous l'effet de la pesanteur en formant une accumulation remplissant le compartiment de mesure en continu, tandis que le fraction restante du courant de produit passe sous la forme d'un courant de dérivation à côté du compartiment de mesure et se réunit, après celui-ci, avec le courant de mesure qui en sort;

— un dispositif (8) pour mesurer la variation de capacité du condensateur ($C_x$; 4, 5) provoquée par le courant de mesure;

— un ensemble électronique (9) de traitement des signaux, destiné à déterminer l'humidité du produit à partir de la variation de capacité;

caractérisé par un dispositif de réglage de niveau (24 à 34) destiné à régler le débit du courant de dérivation sur une hauteur de montée constante dans un canal de dérivation (22) canalisant le courant de dérivation, qui comprend un capteur de valeur instantanée (25) et un organe de réglage (31).

8. Dispositif selon la revendication 7, caractérisé en ce que le compartiment de mesure en continu (1) et le canal de dérivation (22) sont disposés au voisinage immédiat l'un de l'autre et conformés de telle façon que leurs axes longitudinaux sont sensiblement verticaux et mutuellement parallèles, leurs ouvertures d'entrée sont reliées par un canal d'évacuation ou trop-plein (22') commun, et en ce que le canal de dérivation (22) a, sur toute sa longueur, une plus grande section droite que l'ouverture de sortie du compartiment de mesure (1).

9. Dispositif selon l'une au moins des revendications 7 et 8, caractérisé en ce que les sorties du compartiment de mesure (1) et du canal de dérivation (22) débouchent dans une décharge de courant de produit commune (23).

10. Dispositif selon l'une des revendications 7 à 9, caractérisé en ce que, dans le dispositif de réglage de niveau (24 à 34), le capteur de valeur prescrite est une membrane (25) réalisée sous la forme d'une section de paroi du canal de dérivation (22), et l'organe de réglage est un organe de réglage de débit commandant les variations de section droite de la décharge de courant de produit (23).

11. Dispositif selon la revendication 10, caractérisé en ce qu'il comporte, en plus, des moyens (33) manoeuvrés manuellement, pour dégager ou obturer la décharge de courant de produit (23) au moyen de l'organe de réglage de débit (31).

12. Dispositif selon l'une des revendications 7 à 11, caractérisé en ce qu'il comporte un appareil de mesure de débit (7) pour mesurer le débit instantané du courant de produit.

13. Dispositif selon l'une des revendications 7 à 12, caractérisé par un circuit de réglage de débit (71) destiné à régler le débit du courant de produit à une valeur constante.

14. Dispositif selon l'une des revendications 12 et 13, caractérisé en ce qu'en plus du dispositif de mesure de capacité (8), l'appareil de mesure de débit (7) est également relié aux entrées de signaux de l'ensemble électronique de traitement des signaux (9), et l'ensemble électronique de traitement des signaux (9) est relié à un organe de commande (10, 40) comprenant un comparateur destiné à commander un organe de réglage, tel que, par exemple, un appareil de mouillage (19), placé en aval de la décharge de courant de produit (23), dans lequel le courant de produit est amené à une teneur en humidité prédéterminée.

15. Dispositif selon la revendication 14, caractérisé en ce que le compartiment de mesure en continu (1) est réalisé sous la forme d'un récipient de pesée d'un dispositif de pesée (2) dont la sortie de mesure est reliée à l'entrée de l'ensemble électronique (9) de traitement des signaux.

16. Dispositif selon l'une des revendications 7 à 15, caractérisé en ce que la paroi extérieure du compartiment de mesure en continu (1) est réalisée sous la forme d'une première armature (4) du condensateur, et en ce qu'une seconde armature (5) est placée à l'intérieur du compartiment de mesure en continu (1).

**Claims**

1. Process for the continuous capacitive determination of the moisture level of a free-flowing food forming a product flow in the continuous process and in particular a food, whose product constituents are inhomogeneously mixed, in which the product flow is subdivided into a measurement flow and a bypass flow, the measuring flow being continuously guided through a measurement or test cell (1, 1'), having an inlet and an outlet which is smaller than the latter and which is constructed as an electrical capacitor ($C_x$; 4, 5), in such a way that, accompanied by the formation of an accumulation in the measurement cell (1, 1'), it trickles through the latter due to gravity, the capacitance change of capacitor ($C_x$; 4, 5) caused by the measurement flow is continuously measured, the two flows are brought together again behind the measurement cell and the product moisture level is determined from the measured capacitance change, characterized in that the product throughput in the bypass flow is regulated to a substantially constant height of rise in a bypass line (22) carrying the bypass flow.

2. Process according to claim 1, characterized in that the capacitance of the capacitor ($C_x$; 4, 5) is so measured in two cyclically repeating phases that
a) during a first phase a predetermined voltage ($U_{ref}$) is applied to the capacitor ($C_x$; 4, 5) and an additional reference capacitor ($C_{ref}$) is discharged and
b) during a following second phase the charge of the capacitor ($C_x$; 4, 5) is reversed to the reference capacitor ($C_{ref}$) and its voltage ($U_1$) is measured.

3. Process according to claim 2, characterized in that the product moisture level is determined from the mean value of several values of voltage ($U_1$) of reference capacitor ($C_{ref}$).

4. Process according to one of the preceding claims, characterized in that the product is weighed in the measurement cell (1, 1').

5. Process according to one of the preceding claims, characterized in that the instantaneous throughput capacity of the two partial flows brought together again is measured.

6. Process according to claim 5, characterized in that from the determined product moisture level, as well as the instantaneous throughput capacity and a predetermined desired moisture value, the water quantity to be added to the product flow for attaining the desired moisture value is determined and from it is obtained a control signal for controlling the supply of a deficiency water quantity.

7. Apparatus for the continuous capacitive determination of the moisture level of a free-flowing food forming a product flow in a continuous process and in particular a food, whose product constituents are inhomogeneously mixed, with a continuous measuring vessel (1) constructed as a capacitor ($C_x$; 4, 5) and arranged in the path of the product flow and whose outlet is smaller than its inlet and which is arranged and constructed in such a way that part of the product flow forming a measurement flow and accompanied by the formation of an accumulation filling said measuring vessel trickles through the latter due to gravity, whilst the remainder of the product flow is led past the measuring vessel as a bypass flow and is combined behind it with the measurement flow passing out of the same, a device (8) for measuring the capacitance change of capacitor ($C_x$; 4, 5) brought about by the measurement flow and signal processing electronics (9) for determining the product moisture level from the capacitance change, characterized by a level regulating device (24 to 34) for regulating the throughput capacity of the bypass flow to a constant height of rise in a bypass line (22) carrying the bypass flow and which comprises an actual value sensor (25) and a regulating element (31).

8. Apparatus according to claim 7, characterized in that the continuous measuring vessel (1) and the bypass line (22) are arranged and constructed in directly juxtaposed manner in such a way that their longitudinal axes are substantially vertical and parallel to one another, their inlets are connected via a common overflow passage (22') and over its entire length the bypass line (22) has a larger cross-section than the outlet of measuring vessel (1).

9. Apparatus according to at least one of the claims 7 and 8, characterized in that the outlets of the measuring vessel (1) and bypass line (22) issue into a common product flow outlet (23).

10. Apparatus according to one of the claims 7 to 9, characterized in that in the level regulating device (24 to 34), the actual value sensor is a membrane (25) constructed as a wall portion of the bypass line (22) and the regulating element is a flow regulating member (31) controllably modifying the opening cross-section of the product flow outlet (23).

11. Apparatus according to claim 10, characterized in that additionally manually operable means (33) for freeing or closing the product flow outlet (23) by means of the flow regulating member (31) are provided.

12. Apparatus according to one of the claims 7 to 11, characterized by a throughput meter (7) for determining the instantaneous throughput capacity of the product flow.

13. Apparatus according to one of the claims 7 to 12, characterized by a throughput control loop (71) for controlling a constant product flow throughput.

14. Apparatus according to one of the claims 12 or 13, characterized in that, apart from the capacitance measuring device (8), also the throughput meter (7) is connected to the signal inputs of the signal processing electronics (9) and the latter is located on the output side on a control member (10, 40) containing a comparator for controlling a regulating element, such as e.g. a power supply apparatus (19) arranged downstream of the product flow outlet (23) and in which the product flow is brought to a predetermined moisture content.

15. Apparatus according to claim 14, characterized in that the continuous measuring vessel (1) is constructed as the weighting vessel of a weighing apparatus (2), whose measurement output is connected to the input of the signal processing electronics (9).

16. Apparatus according to one of the claims 7 to 15, characterized in that the outer wall of the continuous measuring vessel (1) is constructed as a first coating (4) of the capacitor and a second coating (5) is arranged in the interior of the continuous measuring vessel (1).

*Fig. 1*

Fig. 2

Fig.3

Fig. 4

Fig.5

Fig.6

# Fig. 7